# EUROPEAN PATENT SPECIFICATION

(11) **EP 2 231 121 B1**
(45) Date of publication and mention of the grant of the patent: **03.08.2011**
(21) Application number: 08863025.6
(22) Date of filing: 15.12.2008
(51) Int. Cl.: A61K 9/16, A61K 9/14, A61K 9/20, A61K 9/28, A61K 31/00

(54) **PHARMACEUTICAL COMPOSITIONS**
PHARMAZEUTISCHE ZUSAMMENSETZUNGEN
COMPOSITIONS PHARMACEUTIQUES

(30) Priority: 14.12.2007 GB 0724478
(43) Date of publication of application: 29.09.2010
(73) Proprietor: KARO BIO AB, 141 57 Huddinge (SE)
(72) Inventor: ANDERSSON, Carl-Magnus, S-245 64 Hjärup (SE); QVARNSTRÖM, Johanna, S-141 59 Huddinge (SE); BERGGREN, Jonas, S-753 26 Uppsala (SE); NILSSON, Marita, S-147 71 Grödinge (SE)
(74) Representative: Carter, Caroline Mercedes
(86) International application number: PCT/EP2008/010636
(87) International publication number: WO 2009/077147

(56) References cited:
- EP-A2- 1 291 021
- WO-A1-01/60784
- WO-A1-2007/110226
- BADAWY SHERIF ET AL: "Effect of processing and formulation variables on the stability of a salt of a weakly basic drug candidate" PHARMACEUTICAL DEVELOPMENT AND TECHNOLOGY, NEW YORK, NY, US, vol. 9, no. 3, 1 August 2004 (2004-08-01), pages 239-245, XP009127510 ISSN: 1083-7450

## Description

This invention relates to pharmaceutical compositions. In particular, it relates to pharmaceutical compositions in which the pharmacologically-active ingredients are protected from degradation.

Compound 1A having the following structure: 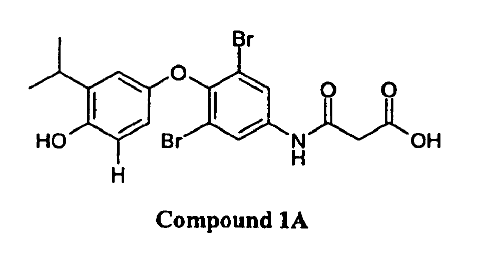 is described in WO 01/60784 (IUPAC name 3-[[3,5-Dibromo-4-[4-hydroxy-3-(1-methylethyl)-phenoxy]-phenyl]-amino]-3-oxopropanoic acid). Compound 1A and a series of related compounds are described as agonists of thyroid hormone receptors, in particular the TRβ receptor. Such compounds should be useful in the treatment or prevention of a disease associated with metabolic dysfunction or which is dependent upon the expression of a triiodothyronine (T₃)-regulated gene. Such diseases include, for example, obesity, hypercholesterolemia, atherosclerosis, cardiac arrhythmias, depression, osteoporosis, hypothyroidism, goitre, thyroid cancer as well as glaucoma and congestive heart failure. Formulations of Compound 1A and related compounds are disclosed in WO 2007/110226.

It has been found that certain compositions containing Compound 1A tend to discolour on prolonged storage indicating degradation of the composition over extended periods. Furthermore, it has been found that significant degradation of the compositions, specifically, degradation of the active ingredient, Compound 1A, occurs rapidly when the compositions are stored at room temperature and refrigeration is necessary if the compositions are to be stored without unacceptable levels of degradation occurring.

According to a first aspect, the present invention provides a pharmaceutical composition suitable for oral administration, comprising an admixture of:
(i) a compound of Formula IA: 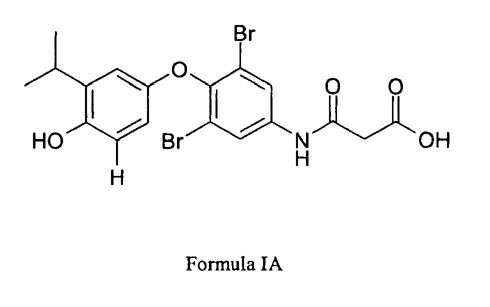 or a pharmaceutically acceptable salt or ester or solvate thereof;
(ii) a pharmaceutically acceptable particulate solid which is a Group 1 or Group 2 metal carbonate or bicarbonate; and
(iii) optionally one or more further pharmaceutical excipients,
wherein component (ii) makes up at least 1% by weight of the admixture.

The compound of formula IA is an example of a compound of Formula I: 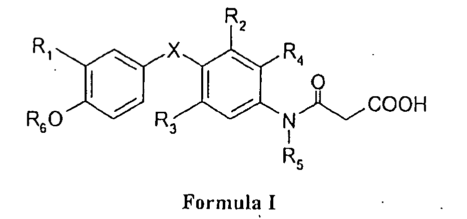 wherein:
R₁ is selected from hydrogen, halogen, trifluoromethyl, C₁₋₆ alkyl, or C₃₋₇ cycloalkyl;
X is oxygen (-O-), or methylene (-CH₂-);
R₂ and R₃ are the same or different and are halogen or C₁₋₄ alkyl;
R₄ is hydrogen or C₁₋₄ alkyl;
R₅ is hydrogen or C₁₋₄ alkyl; and
R₆ is hydrogen, or an alkanoyl or aroyl group, or other group capable of bioconversion to generate the free phenol structure wherein R₆ = HI.

It was surprisingly found that a composition comprising an admixture of a compound of Formula I and at least 1% by weight based on the total weight of the admixture of a pharmaceutically acceptable basic particulate solid, such as calcium carbonate, significantly reduced the degradation of the composition as compared to formulations not comprising such basic particulate solid, particularly compositions such as those disclosed in WO 2007/110226. Compositions comprising compounds of Formula I admixed with calcium carbonate were found to have enhanced shelf stability, especially over extended periods and/or when stored at room temperature. In contrast various other pharmaceutical excipients such as mannitol had a detrimental effect on the stability of the compositions comprising compounds of Formula I. In particular, it has been found that even compounds of Formula I that have been milled during the preparation of a dosage form do not significantly degrade when the pharmaceutical composition includes a pharmaceutically acceptable basic particulate solid, such as calcium carbonate. Degradation has proved to be a significant problem in milled compositions. The term "milled" as employed herein refers to the grinding of a solid in a mill or other grinder to form a fine powder.

A major degradation product of Compound 1A has been found to arise from decarboxylation of the ß-keto carboxylic acid functional group to form an acetamide (Compound 2A). It was found that when a pharmaceutical composition comprising Compound 1A also comprises calcium carbonate the formation of Compound 2A is significantly slowed.

A particulate solid which is an alkali metal (Group 1) or alkaline earth metal (Group 2) metal carbonate or bicarbonate is an example of a basic particulate solid.

The term "basic particulate solid" as used herein refers to a particulate solid which provides an alkaline pH in pure water. The term "acidic" used herein relates to a substance which provides an acidic pH in water. For example, a substance that provides a pH of less than 7.0, preferably a pH of less than 6.5 and especially a.pH of less than 6.0 when dispersed or dissolved in pure water. A neutral substance is a substance that provides a neutral pH in water, that is, a substance that provides a pH of approximately 7.0 when dispersed or dissolved in pure water. The term "filler" as used herein relates to a particulate solid material used to make up the bulk of a pharmaceutical preparation.

Examples of a Group 1 or Group 2 metal carbonate or bicarbonate. include sodium carbonate, potassium carbonate, magnesium carbonate, calcium carbonate, sodium bicarbonate, potassium bicarbonate, magnesium bicarbonate and calcium bicarbonate. Particularly preferred particulate solids which are an alkali metal (Group 1) or alkaline earth metal (Group 2) metal carbonate or bicarbonate include calcium carbonate and magnesium carbonate. A 10% by weight dispersion of calcium carbonate in pure water provides a pH of approximately 8.3. The composition may include more than one particulate solid which is an alkali metal (Group 1) or alkaline earth metal (Group 2) metal carbonate or bicarbonate. Pharmaceutically acceptable particulate solids which are an alkali metal (Group 1) or alkaline earth metal (Group 2) metal carbonate or bicarbonate are present at a level sufficient to attenuate the degradation of the compositions. The specific level which is sufficient to achieve such attenuation is, in part at least, dependent on the overall composition of the tablet or other solid dosage form. In certain matrices, a low loading sufficient to give an excess of said particulate solid over the compound of formula 1A, will suffice, however the use of higher levels of the particulate solid are preferred. Oral dosage forms comprising from 1% are included in the present invention. Preferably, pharmaceutically acceptable particulate solids which are an alkali metal (Group 1) or alkaline earth metal (Group 2) metal carbonate or bicarbonate make up at least 5%, for example at least 10%, for example at least 20%, for example at least 40%, for example at least 50 %, for example at least 60 %, for example at least 70 % by weight of the admixture. In some embodiments, said pharmaceutically acceptable particulate solids make up at least 80 % by weight of the admixture. In other embodiments, said pharmaceutically acceptable particulate solids make up from 10 to 20 % by weight of the admixture. In embodiments in which the composition comprises further components such as a coating in addition to the admixture, the pharmaceutically acceptable particulate solids which are an alkali metal (Group 1) or alkaline earth metal (Group 2) metal carbonate or bicarbonate preferably make up at least 10% by weight, 20% by weight, for example at least 40 %, for example at least 50 %, for example at least 60 % by weight of the entire composition. In some embodiments in which the composition comprises further components in addition to the admixture, pharmaceutically acceptable particulate solids which are an alkali metal (Group 1) or alkaline earth metal (Group 2) metal carbonate or bicarbonate make up at least 70 % by weight of the entire composition for example at least 80% of the entire composition. Preferably the particulate solid which is an alkali metal (Group 1) or alkaline earth metal (Group 2) metal carbonate or bicarbonate is present in the composition as a filler. Fillers other than said pharmaceutically acceptable particulate solids may be present if desired. In one embodiment of the invention, the composition, and in particular the admixture comprising the compound of Formula IA or salt or ester or solvate thereof, is substantially free of any fillers other than said particulate solids, for example acidic or neutral fillers. In another embodiment, the composition and especially the admixture contains one or more neutral fillers.

It was surprisingly found that other common pharmaceutical fillers were not as effective in attenuating the degradation of the compositions as particulate solids which are an alkali metal (Group 1) or alkaline earth metal (Group 2) metal carbonate or bicarbonate. In particular, it was found that mannitol and calcium hydrogen phosphate (e.g. anhydrous) had a detrimental effect on the stability of the compositions. Preferably, the composition, and in particular the admixture comprising the compound of Formula I or A salt or ester or solvate thereof, is substantially free of mannitol, sucrose, glucose, dextrose, lactose, xylitol, fructose, sorbitol, calcium phosphate and/or calcium sulphate. In certain embodiments, the composition of the invention is substantially free of microcrystalline cellulose; in other embodiments, the composition may contain microcrystalline cellulose. For example, the fillers in the admixture may comprise 10-20% weight of basic particulate solid and 80-90% weight microcrystalline cellulose.

The term "substantially free" as used herein when referring to a particular constituent of the admixture means the admixture contains no more than 5% by weight of the constituent based on the total weight of the admixture, preferably no more than 2% by weight, more preferably no more than 1% by weight and especially no more than 0.3% by weight of the constituent based on the total weight of the admixture. For example, the admixture may have no more than 0.1% by weight by weight of the constituent based on the total weight of the admixture and in some embodiments the admixture may have no more than 0.01 % by weight of the constituent based on the total weight of the admixture.

In addition to the impound of formula IA or salt or ester or solvate thereof and the particulate solid, which is an alkali metal (Group 1) or alkaline earth metal (Group 2) metal carbonate or bicarbonate, the admixture of the compositions ester invention may comprise one or more further pharmaceutical excipients. Preferred further pharmaceutical excipients should not have a detrimental effect on the stability of the compound of Formula IA or salt or ester or solvate thereof. The further pharmaceutical excipients are preferably neutral or basic. The one or more further pharmaceutical excipients may comprise one or more of a binder, a disintegrant and a lubricant. Exemplary binders include maltodextrin. Preferably, the binder is maltodextrin. Exemplary disintegrants include salts of carboxymethyl cellulose (the sodium salt being available commercially as Croscarmellose sodium), carboxymethyl starch derivatives (such as the sodium salt that is available commercially as Primojel), and starch (e.g. the starch available commercially as Maydis amylum). Preferably, the disintegrant is carboxymethyl cellulose, a carboxymethyl starch derivatives or starch. Preferably, the lubricant is magnesium stearate. The presence of one or more of maltodextrin, Croscarmellose sodium, Primojel, Maydis amylum and magnesium stearate in the composition has not been found to have a detrimental effect on the stability of the compound of Formula IA. In one embodiment the composition comprises an admixture of a compound of Formula IA, calcium carbonate, maltodextrin, carboxymethylcellulose and magnesium stearate.

Ingredients which may in some embodiments be present in the admixture of the present invention, but which in other embodiments of the invention are preferably absent from the admixture, include cellulose ethers (e.g. the 2-hydroxypropylmethyl ether of starch available under the trade mark Hypromellose 6); mannitol; cellulose; lactose monohydrate; calcium hydrogen phosphate anhydride; dibasic calcium phosphate dehydrate; starch for example pregelatinised starch; gelatine; silica; polyethylene glycol; polyvinyl acetate phthalate; and/or triethyl citrate. Some acidic excipients have been found to have a detrimental effect on the stability of the compositions when admixed with the compound of Formula IA. The admixture may be substantially free of acidic excipients.

The compositions may be formulated in a process in which the active ingredients are mixed with a solvent. Traces of solvent may be present in the compositions.

In certain embodiments of the first aspect of the invention, the composition may include an antioxidant. Suitable antioxidants include, for example, sodium ascorbate. The presence of acidic antioxidants may however be detrimental to the stability of the composition. Therefore, in some embodiments of the invention, the admixture comprising the compound of Formula IA or salt or ester or solvate thereof is substantially free of an antioxidant, especially an acidic antioxidant.

In a further, preferred, embodiment of the invention, the composition is formed into a solid dosage form (e.g. a tablet or capsule) that comprises a core and a coating. Preferably, the dosage form is a tablet. In one embodiment the composition is a coated tablet wherein the admixture of components (i), (ii) and optional components (iii) are present in the core of the tablet. Component (i) may be milled either before or after being admixed with the pharmaceutically acceptable particulate solid which is an alkali metal (Group 1) or alkaline earth metal (Group 2) metal carbonate or bicarbonate.

It has been found that the presence of various excipients can be tolerated in the compositions when those compounds are not admixed with the compound of Formula IA or salt or ester or solvate thereof in the core of the dosage form. For example, it has been found that when various excipients are present as a constituent of the coating they do not have a detrimental effect on the stability of the compound of Formula IA present in the core. For example, triethyl citrate may be present in a coating without adversely affecting the stability of the composition whereas the presence of triethyl citrate in the core of a tablet may enhance the degradation of the composition. The coating may, for example, comprise one or more of methacrylic acid-ethyl acrylate copolymer (1:1), talc (magnesium silicate), triethyl citrate and Opadry® AMB (a coating material available from Colorcon, PA, USA). The presence of stearic acid, Opadry AMB, citric acid and talc in coatings of compositions comprising a core having a compound of Formula IA has not been found to be detrimental to stability.

In one embodiment, the composition is provided with an enteric coating. Preferably, the composition is an enteric coated tablet. The enteric coating is preferably formed using any commercially-available polymer produced for such a purpose. As examples of such polymers, those based on acrylates, methacrylates or copolymers thereof (such as the range of enteric coating polymers marketed under the name Eudragit® by Dcgussa/Roehm), polyvinyl acetate phthalate, cellulose acetate phthalate, hydroxypropylmethylcellulose acetate succinate, hydroxypropylmethylcellulose phthalate, hydroxymethylcelluolse acetate succinate and carboxymethylethylcellulose may be mentioned. Different coatings which dissolve at different pH ranges may be used for different applications. In a particular embodiment, the enteric coating comprises a methacrylic acid-ethyl acrylate copolymer. The constituent monomers of such a copolymer may be present in the ratio 1:1. The enteric coating also preferably contains a glidant component, such as talc. A plasticiser may also be advantageously included. A suitable plasticiser is triethyl citrate.

In most circumstances, it may be preferable for an inert coating to be provided between that portion of the composition containing the compound of Formula IA or salt or ester or solvate thereof and the enteric coating. Preferably, an inert coating is provided between the core of a dosage form and the enteric coating. Enteric coatings are typically composed of acidic polymers and hence, by their very nature, have the potential to lead to deleterious changes in certain active ingredients. Similarly, a basic tablet core can lead to deleterious changes in an enteric coating. An interposed inert coating (made from, for example, a cellulose derivative, such as hydroxypropyl cellulose or hydroxypropylmethyl cellulose) tends to inhibit such interactions. An inert coating is one which is resistant to reaction with ingredients of both the tablet core and the enteric coating. The inert coat should, of course, be soluble (or otherwise dispersible) in the intestinal media in order to allow the active ingredient to be released. Suitable coating materials include PVA-PEG graft copolymers; cellulose ethers; hypromellose phthalate; cellulose acetate phthalate; hypromellose; maltodextrin; polydextrosepolyvinylpyrrolidone; and shellac. Commercially-available materials suitable for use as an inert coat include for example those marketed under the trade marks Aquacoat AS-LG (available from FMC), Kollicoat IR (available from BASF) and Sepifilm.

The term 'enteric coating' as used herein is intended to include coatings applied to a composition/dosage form once the dosage form is otherwise essentially complete and those applied at intermediate stages of dosage form manufacture. Thus, compositions are included in which the compound of Formula IA or salt or ester or solvate thereof is formulated with excipients into granules which are then enteric coated prior to further processing, such as compression into tablets or filling into capsules, for example gelatin capsules. Those skilled in the art will appreciate that the term "enteric coating" further intends to specify a coating which displays specific disintegration and dissolution properties. The present invention specifically concerns such enteric coatings which have dissolution properties which efficiently protect the tablet core ingredients, such as the compound of Formula IA, from exposure to the acidic environment of the GI tract. Coatings which dissolve or otherwise disintegrate or disperse only at pH values in excess of 5 are preferred. Such coatings may dissolve at pH values above 5.5, such as above pH 6, or above pH 6.5. Mixtures of different grades and makes of such enteric coatings are included in the invention.

According to a second aspect, the invention provides a method of preparing a pharmaceutical composition suitable for oral administration, comprising the step of admixing a compound of Formula IA or salt or ester or solvate thereof and a pharmaceutically acceptable particulate solid which is an alkali metal (Group 1) or alkaline earth metal (Group 2) metal carbonate or bicarbonate. Further pharmaceutical excipients may also be admixed with these ingredients. The method may comprise the step of milling the Compound of Formula IA or salt or ester or solvate thereof. The method may comprise the step of milling the admixture, or may comprise the step of milling the Compound of Formula IA or salt or ester or solvate thereof prior to admixing it with said pharmaceutically acceptable particulate solid. The method may include the step of granulating the admixture. The method may comprise the step of providing the granules with a coating (e.g. an inert coating or an enteric coating). The method may further comprise the step of processing the admixture into a solid unit dosage form. The processing of the admixture into the solid unit dosage form may include the step of granulating the admixture. The admixture or granules may be formed into a solid unit dosage form (e.g. a tablet) core. The method may comprise the step of providing the solid unit dosage form core with a coating. Preferably, the method includes the step of providing the solid unit dosage form core with an inert coating. Preferably the method includes the step of providing the solid unit dosage form core with an enteric coating.

The invention further provides a method of preparing a pharmaceutical composition suitable for oral administration, comprising (i) a compound of Formula IA or a pharmaceutically acceptable salt or ester or solvate thereof and (ii) a pharmaceutically acceptable particulate solid which is an alkali metal (Group 1) or alkaline earth metal (Group 2) metal carbonate or bicarbonate comprising the steps of:
a. optionally mixing said pharmaceutically acceptable particulate solid and a binder;
b. dissolving component (i) in a solvent;
c. mixing the solution of component (i) in a solvent from step (b) with component (ii) that has optionally been mixed with the binder in optional step (a) to form a wet mass;
d. optionally granulating the wet mass from step (c);
e. drying the wet mass from step (c) or granulate from optional step (d);
f. optionally grinding or milling or sieving the mixture;
g. optionally adding a disintegrant and mixing;
h. optionally adding a lubricant and mixing;
i. forming the mixture into tablets;
j. optionally coating the tablets with an inert coating; and
k. coating the tablets with an enteric coating.

When the compound of Formula IA is present in the form of an ester, an alkyl ester thereof is preferred, especially a C₁₋₄ alkyl ester.

Preferably the compound of Formula IA is the free acid or a salt, especially the calcium salt. It is believed that the calcium salt of the compound of Formula IA is novel.

When the compound of Formula IA is present in the form of a pharmaceutically acceptable salt, such salts may include metal salts, such as alkali metal or alkaline earth metal salts, for example sodium, potassium, calcium or magnesium salts, or salts with ammonia or an organic amine, such as morpholine, thiomorpholine, piperidine, pyrrolidine, a mono, di or tri-lower alkylamine, for example ethyl, tertbutyl, diethyl, diisopropyl, triethyl, tributyl or dimethylpropylamine, or a mono, di or trihydroxy lower alkylamine for example mono, di or triethanolamine. Preferred salts of the compound of Formula IA include sodium, potassium, calcium and magnesium salts and salts with pharmaceutically acceptable organic amines. The calcium salt is especially preferred. The calcium salt of Compound 1A has been found to be particularly resistant to degradation.

The compound of Formula IA may of course be solvated if desired, for example hydrates may be used in the present invention.

The present invention also provides a composition according to the invention, for use in therapy. Specifically, the invention finds utility in preventing, inhibiting or treating a disease associated with metabolism dysfunction, or which is dependent on the expression of a triiodothyronine (T₃)-regulated gene. The disease may be selected from obesity, hypercholesterolemia, dyslipidaemia, atherosclerosis, cardiac arrhythmias, depression, osteoporosis, hypothyroidism, goitre, thyroid cancer as well as glaucoma and congestive heart failure.

In the use described immediately above, the medicament or composition may be administered at a dosing interval of from 30 minutes to one month. More preferably, the dosing interval is from one to seven days, even more preferably one to three days. The typical adult human dose range for compounds (i) would be around 1µg to around 2000 µg per day. For many compounds (i), the daily dose would be less than 300 µg. Preferably, the dose of compound (i) is from around 1 µg to around 200 µg per day, more preferably around 1 to around 100 µg per day. For example, the compounds (i) may be administered in one dose, two doses, three doses or four doses per day. Preferably, the amount of compound (i) per unit dose of composition is from 1 to 200 µg, more preferably 1 to 100 µg, more preferably 1, 5, 10, 20, 25 or 50 µg. For example, the amount of compound (i) per unit dose may be from 10 to 100, for example from 20 to 80, typically from 25 to 50, µg.

The composition according to the invention may also comprise a further pharmacologically active ingredient selected from hypolipidaemic agents, including statins, for example atorvastatin or simvastatin, antidiabetic agents, antidepressants, bone resorption inhibitors, appetite suppressants and/or anti-obesity agents. In a particularly preferred embodiment, the composition according to the invention may also comprise the anti-hyperlipidaemic agent ezetimibe.

The further pharmacologically active ingredients tend to have additive or synergistic effects with the compound of Formula IA or salts esters or solvates thereof so as to enhance the metabolic effects thereof. Therefore, the present invention finds utility as combination medicament suitable for oral administration, comprising: .
(1) a first pharmaceutical composition comprising the compound of Formula IA or a pharmaceutically acceptable salt or ester or solvate thereof, in admixture with a pharmaceutically acceptable particulate solid which is a an alkali metal (Group 1) or alkaline earth metal (Group 2) metal carbonate or bicarbonate and as described above; and
(2) a second pharmaceutical composition comprising at least one antioxidant, wherein the second pharmaceutical composition contains at least one pharmaceutically-acceptable excipient, and wherein the first and second pharmaceutical compositions may be administered simultaneously, sequentially or separately.

The combination medicament of the present invention takes advantage of the fact that, as described in WO 2007/110226, it is desirable that an antioxidant should be present when the compound of formula IA or a salt or ester or solvate comes into contact with acid and a source of nitrite. Provided the two compositions of the combination medicament are given in such temporal proximity that there is overlap between their periods of residence in the stomach, the active compound should enjoy at least a degree of stabilisation against the nitration reaction.

The invention will now be described in more detail by way of example only and with reference to the appended drawings, of which:

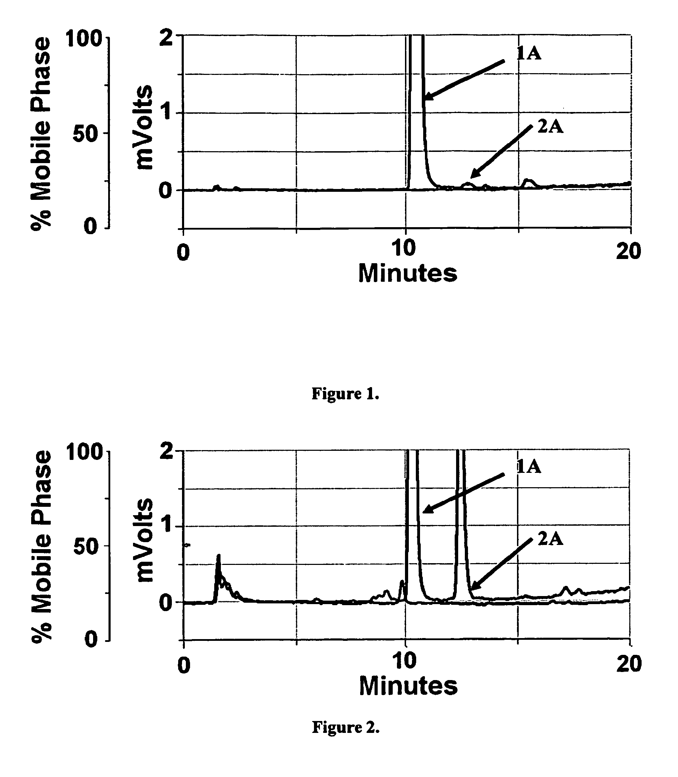

Figure 1 shows an HPLC trace of freshly prepared sample of Compound 1A;
Figure 2 shows an HPLC trace of sample 1 of Example 1 after storage for 4 weeks;

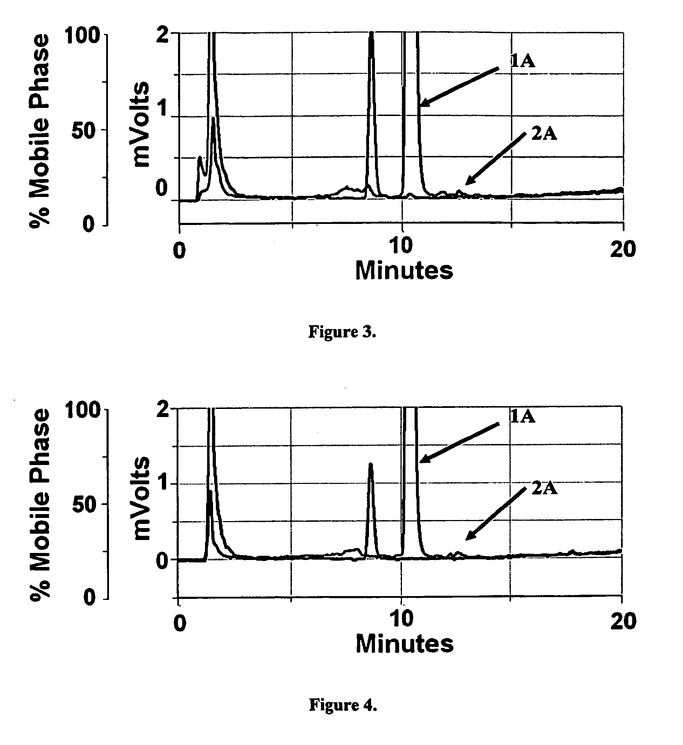

Figure 3 shows an HPLC trace of sample 2 of Example 1 after storage for 4 weeks;
Figure 4 shows an HPLC trace of sample 3 of Example 1 after storage for 4 weeks;

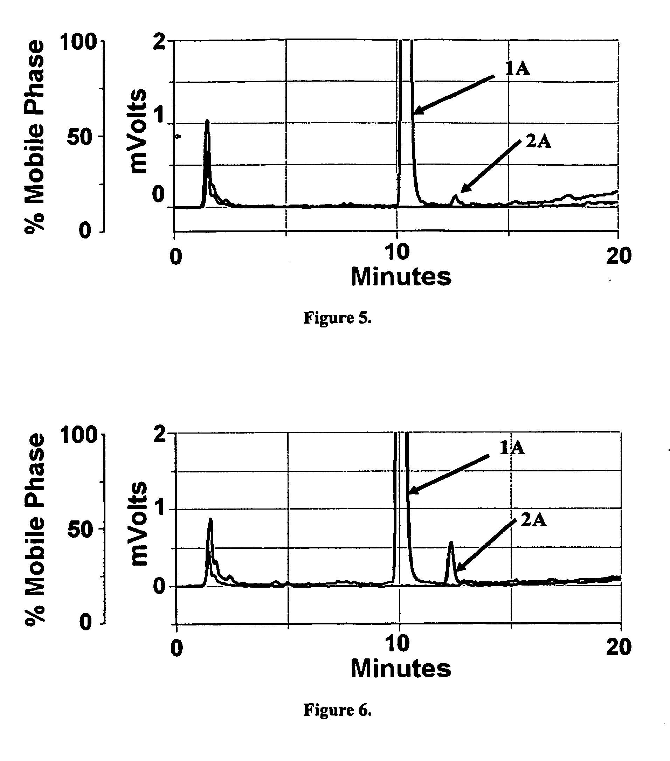

Figure 5 shows an HPLC trace of sample 4 of Example 1 after storage for 4 weeks;
Figure 6 shows an HPLC trace of sample 5 of Example 1 after storage for 4 weeks;

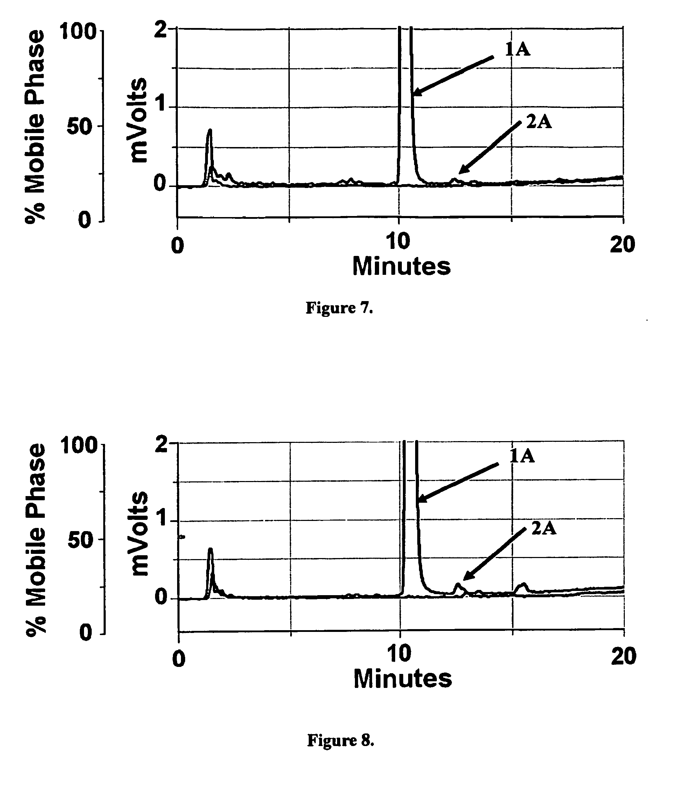

Figure 7 shows an HPLC trace of sample 6 of Example 1 after storage for 4 weeks;
Figure 8 shows an HPLC trace of sample 7 of Example 1 after storage for 4 weeks;

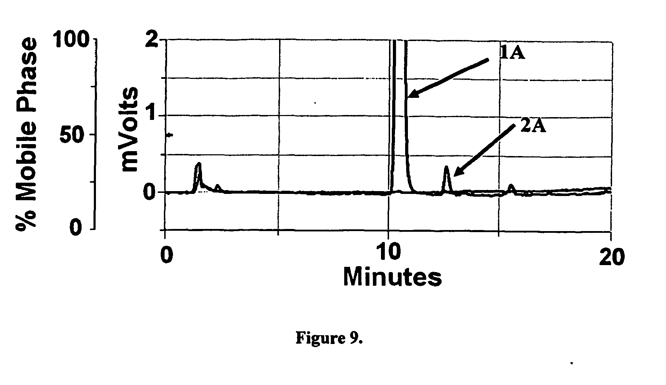

Figure 9 shows an HPLC trace of sample 8 of Example 1 after storage for 4 weeks.

The following materials are used in the Examples and/or are referred to above:
Croscarmellose sodium = CAS 74811-65-7, Cellulose, carboxymethyl ether, sodium salt, crossliked
Primojel = CAS 9063-38-1, Sodium carboxymethyl starch
Maydis amylum = CAS 9005-25-8, Starch
Maltodextrin = CAS 9050-36-6, maltodextrin
Magnesium stearate = CAS 557-04-0, Octadecanoic acid magnesium
Hypromellose = CAS 9004-65-3, Cellulose, 2-hydroxypropyl methyl ether
Mannitol = CAS 69-65-8, D-Mannitol
Cellulosum microcrist = CAS 9004-34-6, Cellulose
Lactose monohydrate = CAS 64044-51-5, *O*-β-D-Galactopyranosyl-(1→4)-α-D-glucopyranose monohydrate, the anhydrous form being CAS 63-42-3 *O*-β-D-Galactopyranosyl-(1→4)-α-D-glucopyranose anhydrous
Pregel starch = CAS 9005-25-8, Pregelatinized starch
Opadry AMB = a water soluble coating material available from Colorcon, PA, USA
Eudragit L30-D50 = a methacrylic acid -ethyl acrylate copolymer (1:1) that is available from Degussa/Roehm GmbH & Co. KG of Darmstadt, Germany Ready having a Ph of 2.1 - 3.0 determined according Ph. Eur. 2.2.3.

### Example 1- Compatibly of Compound IA when mixed with various excipients

### 1.1 - Introduction

The aim of this study is to demonstrate the stability of the compound of Formula IA when dissolved in a solvent and mixed with various excipient blends.

### 1.2 - Sample Preparation

Comparative sample 1 and samples 2 to 6 were prepared by dissolving an unmilled 1 mg sample of Compound 1A in a 1:1 mixture of ethanol and water, adding the solution to the a dry mixture of the excipients listed in Table 1 and mixing with a spatula. Sample 7 was prepared by dissolving a milled 1 mg sample of Compound 1A in a 1:1 mixture of ethanol and water, adding the solution to the a dry mixture of the excipients listed in Table 1 and mixing with a spatula.

Sample 8 was prepared by dissolving an unmilled 1 mg sample of Compound 1A in ethanol, adding the solution to the a dry mixture of the excipients listed in Table 1 and mixing with a spatula.

**Table 1. Guidance amounts of excipients in the granulate compatibility study**

| No. | Solvent (µl) | Hypromellose (mg) | Cellosum microcr. (mg) | Maltodextrin (mg) | Ca carbonate (mg) | Cros-cannellose (mg) | Primojel (mg) | Maydus amylum (mg) | Stacrh pregelatinised (mg) | Granulate¹ (mg) |
|---|---|---|---|---|---|---|---|---|---|---|
| 1 | 280 | 7 | 1579 | | | | | | | |
| 2 | 280 | | | 111 | 1092 | 140 | | | | |
| 3 | 280 | | | 111 | 1092 | | 140 | | | |
| 4 | 280 | | | 111 | 1092 | | | 140 | | |
| 5 | 280 | | | 111 | 1092 | | | | 140 | |
| 6 | 280 | | | | | | | | | 1300 |
| 7 | 0² | | | | | | | | | 1300 |
| 8 | 280³ | | | | | | | | | 1300 |

| | | | | | | | | | | |
|---|---|---|---|---|---|---|---|---|---|---|
| 1.) Granulate contains calcium carbonate with 9% maltodextrin. 2.) Dry mixture with milled Compound 1A. 3.) Compound 1A dissolved in 280 µl ethanol, no water added. | | | | | | | | | | |

Samples 1 to 8 were dried in a tray drier at 40 °C overnight to remove the liquid and then stored in open vials at 50 °C and 75% relative humidity. The samples were then removed after two or four weeks and stored at room temperature for up to four days prior to being analysed.

The blends of Compound 1A and excipients were transferred to a glass volumetric flask and diluted to a volume of 10 ml with a 0.05% trifluoroacetic acid (TFA) solution in 50/50 acetonitrile/water to provide a 1 mg/ml solution of Compound 1A. The solution was stirred using a magnetic stirrer for 20 minutes then left for 5 minutes before 1 ml aliquots were taken using a plastic syringe. The aliquots were filtered through a 0.45 µm syringe filter int amber glass vials for HPLC analysis.

### 1.3 - Results

Results from HPLC analysis of samples stored for 2 and 4 weeks are shown in Table 2 below. The primary degradation product detected is Compound 2A and therefore the area of the trace for degradation product Compound 2A was compared with the area of the trace for compound 1A. Compound 2A is the acetamide formed when Compound 1A is decarboxylated.

**Table 2. Results from the granulate compatibility study with Compound 1A.**

| | | Area % Compound 2A/Compound 1A¹ | |
|---|---|---|---|
| Sample No. | Excipients in granulate blend | 2 weeks | 4 weeks |
| 1 | Hypromellose+cellulosum microcrist (Comparative) | 10.0 | 21 |
| 2 | Maltodextrin+calcium carbonate+croscarmellose | 0.2 | 0.2 |
| 3 | Maltodextrin+calcium carbonate+primojel | 0.2 | 0.2 |
| 4 | Maltodextrin+calcium carbonate+maydis amylum | 0.2 | 0.3 |
| 5 | Maltodextrin+calcium carbonate+pregel starch | 0.8 | 1.4 |
| 6 | Granulate with calcium carbonate and 9% maltodextrin | 0.1 | 0.2 |
| 7 | Granulate with calcium carbonate and 9% maltodextrin- dry mixture² | 0.1 | 0.2 |
| 8 | Granulate with calc. carbonate and 9% maltodextrin, Compound 1A in 100 % EtOH | 0.9 | 1.4 |

| | | | |
|---|---|---|---|
| 1) Initial concentration of Compound 1A is 0.2% 2) 1 mg milled Compound 1A added to the mixture | | | |

In order for the final drug product to be acceptable, the concentration of degradation Compound 2A should be below or equal to 2.0 area % of Compound 1A and especially below or equal to 1.5 area % of Compound 1 A.

Samples 2, 3, 4 and 6 in which Compound 1A was dissolved in ethanol/water 1:1 and mixed with the "wet" blends showed particularly low levels of degradation. Comparative sample 1 demonstrated that Compound 1A was not stable in combination with hypromellose and cellulosum microcrist.

### 1.5 - Conclusions

The results from this study show that Compound 1A is stable when dissolved in ethanol/water 1:1 and granulated with calcium carbonate.

The presence of a pharmaceutically acceptable basic particulate solid in admixture with the compound of Formula 1A may provide conditions under which decarboxylation to form Compund 2A is less favourable. It is also speculated that the basic particulate solid, and calcium carbonate in particular, may affect the crystal structure of the compound of Formula IA resulting in a form in which decarboxylation reactions are less favoured.

### Example 2 - Stability of Compound 1A when milled

### 2.1- Introduction

Investigate stability of milled and unmilled Compound 1A in combination with calcium carbonate.

### 2.2- Sample Preparation

Samples containing 1092 mg of calcium carbonate and 1 of Compound 1A (either milled or unmilled) were prepared and weighed into small amber glass bottles.Samples were stored in open vials at 50 °C and 75% relative humidity. The samples were then removed after two or four weeks and stored at room temperature for up to four days prior to being analysed and were stored in open vials at 50°C and 75% RH. The blends of Compound 1A and calcium carbonate were transferred to a glass volumetric flask and diluted to a volume of 10 ml or 100 ml with a 0.05% trifluoroacetic acid (TFA) solution in 50/50 acetonitrile/water to provide a 1 mg/ml solution of Compound 1A. The solution was stirred using a magnetic stirrer for 20 minutes then left for 5 minutes before 1 ml aliquots were taken using a plastic syringe. The aliquots were filtered through a 0.45 µm syringe filter into amber glass vials for HPLC analysis.

### 2.3 - Results

No difference in stability has been detected between samples of Compound 1A mixed with calcium carbonate that were milled and unmilled either initially or after storage in open vials at 50 °C and 75% relative humidity for 2 or 4 weeks. The area % of Compound 2A compared to Compound 1A remained at the initial concentration of 0.2 in both milled and unmilled samples after 2 and 4 weeks.

### Example 3 - Enterically coated tablets comprising Compound 1A

Tablet cores comprising 25 or 50 µg of Compound 1A in admixture with calcium carbonate were successfully provided with an enteric coating comprising a Methacrylic acid-ethyl acrylate copolymer using a coating pan. No discoloration of the enterically coated tablets was observed on storage at room temperature for four weeks.

### Example 4. Enteric-coated Tablets with an Interposed Inert Layer

In this example, all quantities are given in mg per dosage unit. Calcium carbonate (filler, 122.8) and maltodextrin (binder, 12.15) were dry mixed in a high shear mixer. Compound 1A (0.025) was dissolved, using a mixer, in a blend of purified water (15) and ethanol (10), and the solution was added with agitation to the mixture of calcium carbonate and maltodextrin. The resulting wet mass was granulated, dried using a fluid bed drier, and sieved through a mill. The sieved granulate was then mixed with croscarmellose sodium (disintegrant) (4.2) using a double-cone blender, and the resulting mixture mixed together with magnesium stearate (lubricant, 0.85) in a double-cone blender. The resulting powder blend was compressed into tablets using a rotary tablet press.

An inert coating of Opadry AMB® (6.0) was applied to the resulting tablet cores using a coating pan. A further, enteric, coating was applied by using a 30% dispersion of Eudragit L30-D55®, a 1:1 methacrylic acid-ethyl acrylate copolymer (25.0), together with talc (glidant, 7.0) and triethyl citrate (plasticizer, 1.4) in a coating pan.

A second batch of tablets was prepared in exactly the same way, save that the content of Compound 1A was 0.050 mg/unit.

Satisfactory stable tablets were obtained.

Long-term storage tests were carried out on the enteric coated tablets containing 50 µg of Compound 1A. Normal variation of the initial content of Compound 1A is 47.5-52.5. After storage for 9 months at 30°C and 65% relative humidity, the measured content of Compound 1A was 48.8. No change in the visual appearance of the tablets was observed.

## Claims

1. A pharmaceutical composition suitable for oral administration, comprising an admixture of:
(i) a compound of Formula IA: 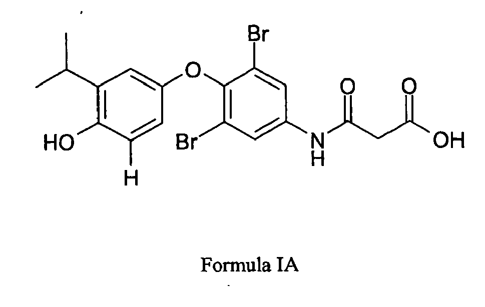 or a pharmaceutically acceptable salt or ester or solvate thereof;
(ii) a pharmaceutically acceptable particulate solid which is a alkaline metal (Group 1) or alkaline earth metal (Group 2) carbonate or bicarbonate; and
(iii) optionally one or more further pharmaceutical excipients,
wherein component (ii) makes up at least 1% by weight of the admixture.

2. The composition according to claim 1, wherein component (ii) makes up at least 40% by weight of the admixture.

3. The composition according to either claim 1 or claim 2, wherein the compound of formula IA is present as the calcium salt.

4. The composition according to any preceding claim, wherein component (ii) is calcium carbonate.

5. The composition accordingly to any preceding claim wherein component (ii) makes up at least 70 % by weight of the admixture.

6. The composition according to any preceding claim, wherein the admixture is substantially free of any fillers other than basic particulate solids.

7. The composition according to any preceding claim wherein component (iii) comprises one or more of a binder, a disintegrant and a lubricant.

8. The composition according to claim 7 wherein the binder is maltodextrin; the disintegrant is a carboxymethylated cellulose, a carboxymethyl starch or a starch; and/or the lubricant is magnesium stearate.

9. The composition according to claim 1, wherein the composition includes sodium ascorbate.

10. The composition according to any preceding claim comprising a core and a coating wherein the admixture of components (i) and (ii) and optional component(s) (iii) is present in the core.

11. The composition according to claim 10 comprising an enteric coating and optionally also comprising an inert coating interposed between the core and the enteric coating.

12. The composition according to any preceding claim also comprising a further pharmacologically active ingredient selected from hypolipidaemic agents, antidiabetic agents, antidepressants, bone resorption inhibitors, appetite suppressants and/or anti-obesity agents.

13. A method of preparing a pharmaceutical composition suitable for oral administration, comprising admixing components (i) and (ii) as defined in any one of claims 1 to 6.

14. The method according to claim13 including the step of forming the admixture of (i) and (ii) into a tablet core and providing the tablet core with an enteric coating.

15. The method according to claim 13 comprising the steps of:
a. optionally mixing component (ii) and a binder;
b. dissolving component (i) in a solvent;
c. mixing the solution of component (i) in a solvent from step (b) with component (ii) that has optionally been mixed with the binder in optional step (a) to form a wet mass;
d. optionally granulating the wet mass from step (c);
e. drying the wet mass from step (c) or granulate from optional step (d);
f. optionally milling the mixture;
g. optionally adding a disintegrant and mixing;
h. optionally adding a lubricant and mixing;
i. forming the mixture into tablets;
j. optionally coating the tablets with an inert coating; and
k. coating the tablets with an enteric coating.

16. A composition according to any of claims 1 to 12, for use in therapy.

17. A composition according to claim 16 for use in preventing, inhibiting or treating a disease associated with metabolism dysfunction.

18. A composition according to claim 16 for use in preventing, inhibiting or treating a disease selected from obesity, hypercholerolemia, dyslipidemia, atherosclerosis, cardiac arrhythmias, depression, osteoporosis, hypothyroidism, goitre, thyroid cancer, glaucoma and congestive heart failure.

## Patentansprüche

1. Pharmazeutische Zusammensetzung, die für die orale Verabreichung geeignet ist, die eine Beimischung von folgenden Substanzen aufweist:
(i) eine Verbindung der Formel IA: 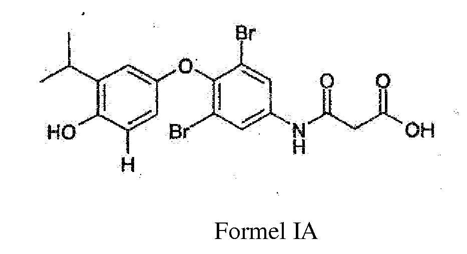 oder ein pharmazeutisch akzeptables Salz oder einen pharmazeutisch akzeptablen Ester oder ein pharmazeutisch akzeptables Solvat davon;
(ii) einen pharmazeutisch akzeptablen, aus Teilchen bestehenden Feststoff, der ein Carbonat oder Bicarbonat eines Alkalimetalls (Gruppe 1) oder eines Erdalkalimetalls (Gruppe 2) ist; und
(iii) gegebenenfalls einen oder mehrere weitere pharmazeutische Träger,
wobei der Bestandteil (ii) mindestens 1 Gew.-% der Beimischung ausmacht.

2. Zusammensetzung nach Anspruch 1,
wobei der Bestandteil (ii) mindestens 40 Gew.-% der Beimischung ausmacht.

3. Zusammensetzung nach Anspruch 1 oder Anspruch 2,
wobei die Verbindung der Formel IA als Calciumsalz vorliegt.

4. Zusammensetzung nach einem der vorstehenden Ansprüche,
wobei der Bestandteil (ii) Calciumcarbonat ist.

5. Zusammensetzung nach einem der vorstehenden Ansprüche,
wobei der Bestandteil (ii) mindestens 70 Gew.-% der Beimischung ausmacht.

6. Zusammensetzung nach einem der vorstehenden Ansprüche,
wobei die Beimischung, abgesehen von basischen, aus Teilchen bestehenden Feststoffen, im wesentlichen frei von irgendwelchen Füllstoffen ist.

7. Zusammensetzung nach einem der vorstehenden Ansprüche,
wobei der Bestandteil (iii) eine oder mehrere Substanzen in Form eines Bindemittels, eines Sprengmittels und eines Gleitmittels aufweist.

8. Zusammensetzung nach Anspruch 7,
wobei das Bindemittel Maltodextrin ist, das Sprengmittel eine carboxymethylierte Cellulose, eine Carboxylmethylstärke oder eine Stärke ist; und/oder das Gleitmittel Magnesiumstearat ist.

9. Zusammensetzung nach Anspruch 1,
wobei die Zusammensetzung Natriumascorbat aufweist.

10. Zusammensetzung nach einem der vorstehenden Ansprüche,
die einen Kern und einen Überzug aufweist, wobei die Beimischung der Bestandteile (i) und (ii) und des (der) wahlfreien Bestandteils (Bestandteile) (iii) in dem Kern vorliegt.

11. Zusammensetzung nach Anspruch 10,
die einen enterischen Überzug und gegebenenfalls auch einen inerten Überzug aufweist, der sich zwischen dem Kern und dem enterischen Überzug befindet.

12. Zusammensetzung nach einem der vorstehenden Ansprüche,
die außerdem einen weiteren pharmakologischen Wirkstoff aufweist, der aus hypolipidämischen Mitteln, antidiabetischen Mitteln, Antidepressiva, Knochenresorptionsinhibitoren, Appetitszüglern und/oder Mitteln gegen Fettsucht ausgewählt ist.

13. Verfahren zum Herstellen einer pharmazeutischen Zusammensetzung, die für die orale Verabreichung geeignet ist,
welches das Beimischen der Bestandteile (i) und (ii) nach einem der Ansprüche 1 bis 6 aufweist.

14. Verfahren nach Anspruch 13,
das einen Schritt zum Formen der Beimischung von (i) und (ii) zu einem Tablettenkern und zum Versehen des Tablettenkerns mit einem enterischen Überzug aufweist.

15. Verfahren nach Anspruch 13,
das die folgenden Schritte aufweist:
a.) wahlfreies Mischen des Bestandteils (ii) und eines Bindemittels;
b.) Lösen des Bestandteils (i) in einem Lösungsmittel;
c.) Mischen der Lösung des Bestandteils (i) in einem Lösungsmittel aus dem Schritt (b) mit dem Bestandteil (ii), der gegebenenfalls im wahlfreien Schritt (a) mit dem Bindemittel gemischt worden ist, um eine feuchte Masse zu bilden;
d.) wahlfreies Granulieren der feuchten Masse aus dem Schritt (c);
e.) Trocknen der feuchten Masse aus dem Schritt (c) oder des Granulats aus dem wahlfreien Schritt (d);
f.) wahlfreies Mahlen des Gemischs;
g.) wahlfreies Zugeben eines Sprengmittels und Mischen;
h.) wahlfreies Zugeben eines Gleitmittels und Mischen;
i.) Formen des Gemischs zu Tabletten;
j.) wahlfreies Überziehen der Tabletten mit einem inerten Überzug; und
k.) Überziehen der Tabletten mit einem enterischen Überzug.

16. Zusammensetzung nach einem der Ansprüche 1 bis 12 für die Verwendung bei der Therapie.

17. Zusammensetzung nach Anspruch 16
für die Verwendung bei der Verhinderung, Hemmung oder Behandlung einer Erkrankung, die mit einer Funktionsstörung des Stoffwechsels verbunden ist.

18. Zusammensetzung nach Anspruch 16
für die Verwendung bei der Verhinderung, Hemmung oder Behandlung einer Erkrankung, die aus Fettsucht, Hypercholesterinämie, Dyslipidämie, Atherosklerose, Herzrhythmusstörung, Depression, Osteoporose, Hypothyreose, Struma, Schilddrüsenkrebs, Glaukom und einem kongestiven Herzfehler ausgewählt ist.

## Revendications

1. Composition pharmaceutique appropriée pour une administration par voie orale, comprenant un mélange de :
(i) un composé de formule IA : 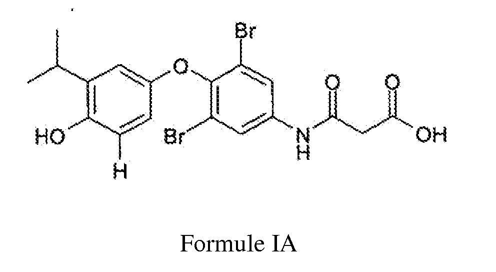 ou un sel ou ester ou solvate pharmaceutiquement acceptable de celui-ci;
(ii) un solide particulaire pharmaceutiquement acceptable qui est un carbonate ou bicarbonate de métal alcalin (groupe 1) ou métal alcalino-terreux (groupe 2) ; et
(iii) éventuellement un ou plusieurs autres excipient pharmaceutiques,
où le composant (ii) représente au moins 1 % en poids du mélange.

2. Composition selon la revendication 1, où le composant (ii) représente au moins 40% en poids du mélange.

3. Composition selon l'une des revendications 1 ou 2, où le composé de formule IA est présent sous forme de sel de calcium.

4. Composition selon l'une quelconque des revendications précédentes, où le composant (ii) est du carbonate de calcium.

5. Composition selon l'une quelconque des revendications précédentes, où le composant (ii) représente au moins 70 % en poids du mélange.

6. Composition selon l'une quelconque des revendications précédentes, où le mélange est sensiblement dépourvu de charges quelconque autres que des solides particulaires basiques.

7. Composition selon l'une quelconque des revendications précédentes, où le composant (iii) comprend un ou plusieurs parmi un liant, un désintégrant et un lubrifiant.

8. Composition selon la revendication 7, où le liant est de la maltodextrine; le désintégrant est une cellulose carboxyméthylée, un carboxyméthyle amidon ou un amidon ; et/ou le lubrifiant est du stéarate de magnésium.

9. Composition selon la revendication 1, où la composition comprend de l'ascorbate de sodium.

10. Composition selon l'une quelconque des revendications précédentes comprenant un noyau et un revêtement, où le mélange des composants (i) et (ii) et du ou des composants éventuels (iii) est présent dans le noyau.

11. Composition selon la revendication 10 comprenant un revêtement entérique et comprenant également éventuellement un revêtement inerte intercalé entre le noyau et le revêtement entérique.

12. Composition selon l'une quelconque des revendications précédentes comprenant également un autre ingrédient pharmacologiquement actif choisi parmi des agents hypolipidémiants, des agents antidiabétiques, des antidépresseurs, des inhibiteurs de résorption osseuse, des inhibiteurs d'appétit et/ou des agents anti-obésité.

13. Procédé de préparation d'une composition pharmaceutique appropriée pour une administration par voie orale, comprenant le mélange de composants (i) et (ii) tel que défini dans l'une quelconque des revendications 1 à 6.

14. Procédé selon la revendication 13 comprenant l'étape consistant à former le mélange de (i) et (ii) en un noyau de comprimé et à munir le noyau de comprimé d'un revêtement entérique.

15. Procédé selon la revendication 13 comprenant les étapes consistant à:
a. mélanger éventuellement le composant (ii) et un liant;
b. dissoudre le composant (i) dans un solvant;
c. mélanger la solution du composant (i) dans un solvant provenant de l'étape (b) avec un composant (ii) qui a été éventuellement mélangé avec le liant dans une étape optionnelle (a) pour former une masse humide;
d. granuler éventuellement la masse humide provenant de l'étape (c)
e. sécher la masse humide provenant de l'étape (c) ou le granulat provenant de l'étape optionnelle (d) ;
f. broyer éventuellement le mélange;
g. ajouter éventuellement un désintégrant et mélanger;
h. ajouter éventuellement un lubrifiant et mélanger;
i. former le mélange en comprimés;
j. revêtir éventuellement les comprimés avec un revêtement inerte; et
k. revêtir les comprimés avec un revêtement entérique.

16. Composition selon l'une quelconque des revendications 1 à 12, destinée à une utilisation en thérapie.

17. Composition selon la revendication 16 destinée à une utilisation pour prévenir, inhiber ou traiter une maladie associée à un dysfonctionnement du métabolisme.

18. Composition selon la revendication 16 destinée à une utilisation pour prévenir, inhiber ou traiter une maladie choisie parmi l'obésité, l'hypercholestérolémie, la dyslipidémie, l'athérosclérose, l'arythmie cardiaque, la dépression, l'ostéoporose, l'hypothyroïdie, le goitre, le cancer de la thyroïde, le glaucome et l'insuffisance cardiaque congestive.
